# FASCICULE DE BREVET EUROPEEN

(11) **EP 1 877 032 B1**
(45) Date de publication et mention de la délivrance du brevet: **25.07.2018**
(21) Numéro de dépôt: 06764577.0
(22) Date de dépôt: 28.04.2006
(51) Int. Cl.: A61K 8/31, A61Q 5/08, A61K 8/81, A61K 8/90, A61K 8/92, A61K 8/06, A61K 8/22

(54) **EMULSION INVERSE COMPRENANT UNE SOLUTION AQUEUSE DE PEROXYDE D'HYDROGENE ET UNE PHASE INERTE DE SOLUBILITE DANS L'EAU INFERIEURE A 1 %**
UMKEHREMULSION MIT EINER WÄSSRIGEN WASSERSTOFFPEROXIDLÖSUNG UND EINER TRÄGHEITSPHASE MIT EINER WASSERLÖSLICHKEIT VON WENIGER ALS 1 %
INVERT EMULSION COMPRISING AN AQUEOUS SOLUTION OF HYDROGEN PEROXIDE AND AN INERT PHASE WITH WATER SOLUBILITY OF LESS THAN 1 %

(30) Priorité: 29.04.2005 FR 0551118; 08.06.2005 US 688351 P
(43) Date de publication de la demande: 16.01.2008
(73) Titulaire: L'Oréal, 75008 Paris (FR)
(72) Inventeur: KRAVTCHENKO, Sylvain, Lu Wan district 200021 Shanghai (CN); DUBIEF, Claude, F-78150 Le Chesnay (FR); NICOLAS-MORGANTINI, Luc, F-60810 Rully (FR)
(74) Mandataire: Prevel, Estelle Nicole
(86) Numéro de dépôt international: PCT/FR2006/000973
(87) Numéro de publication internationale: WO 2006/117469

(56) Documents cités:
- EP-A- 1 291 006
- EP-A- 1 430 875
- US-A1- 2004 235 700

## Description

La présente invention a pour objet une émulsion inverse pour la décoloration des fibres kératiniques, et en particulier des fibres kératiniques humaines telles que les cheveux.

Lorsqu'une personne souhaite changer radicalement de couleur de cheveux, notamment lorsqu'elle souhaite obtenir une couleur plus claire que sa couleur d'origine, il est souvent nécessaire de procéder à une décoloration des cheveux. Pour ce faire, on utilise des produits de décoloration. Cette étape de décoloration est éventuellement associée à une étape de coloration des cheveux.

Il est connu de décolorer les fibres kératiniques, et en particulier les fibres kératiniques humaines telles que les cheveux, avec des compositions de décoloration contenant un ou plusieurs agents oxydants. Parmi les agents oxydants classiquement utilisés, on peut citer le peroxyde d'hydrogène, les composés susceptibles de produire le peroxyde d'hydrogène par hydrolyse tels que le peroxyde d'urée, ou les persels comme les perborates, les percarbonates et les persulfates, le peroxyde d'hydrogène et les persulfates étant particulièrement préférés.

Les compositions de décoloration se présentent principalement sous forme de produits anhydres, poudres ou crèmes, contenant des composés alcalins tels que les amines ou les silicates alcalins, et un réactif peroxygéné tels que les persulfates, les perborates ou les percarbonates, d'ammonium ou de métaux alcalins, que l'on dilue au moment de l'emploi avec une composition aqueuse de peroxyde d'hydrogène.

Les compositions de décoloration peuvent aussi résulter du mélange, au moment de l'emploi, d'une poudre anhydre contenant le réactif peroxygéné avec une composition aqueuse contenant les composés alcalins et une autre composition aqueuse contenant le peroxyde d'hydrogène.

Pour obtenir un produit de décoloration des fibres kératiniques qui soit plus efficace en terme d'éclaircissement et / ou de rapidité, il est en théorie possible d'augmenter la concentration en peroxyde d'hydrogène dans la composition aqueuse de peroxyde d'hydrogène. Cependant, une forte concentration en peroxyde d'hydrogène peut entraîner une dégradation des fibres kératiniques et éventuellement une irritation de la peau. De façon conventionnelle, dans des compositions classiques de décoloration, la concentration en peroxyde d'hydrogène est donc limitée à 12 % en poids, voire à 6 % en poids.

Par ailleurs, la demande de brevet EP 0 193 471 propose de décolorer les cheveux avec une solution anhydre de peroxyde d'hydrogène dans un solvant organique.

Il a aussi été proposé, dans la demande de brevet WO 03/011216, une composition pour la décoloration des cheveux qui se présente sous forme d'une émulsion directe ou inverse renfermant une solution aqueuse de peroxyde d'hydrogène et une phase organique comprenant au moins un composé perfluoré.

Toutefois, les résultats obtenus en terme d'éclaircissement ne sont pas encore satisfaisants.

Le but de la présente invention est de fournir de nouveaux produits d'éclaircissement qui soient plus efficaces que les produits d'éclaircissement connus de l'art antérieur tout en limitant la dégradation des fibres kératiniques et l'irritation de la peau.

Ce but est atteint avec la présente invention qui a pour objet une émulsion inverse pour la décoloration des fibres kératiniques comprenant une phase inerte comprenant au moins un composé liquide non oxygéné et non perfluoré présentant une solubilité dans l'eau à 25 °C inférieure à 1 % et une solution aqueuse de peroxyde d'hydrogène, la phase inerte représentant au moins 20 % en poids du poids total de l'émulsion inverse et le diamètre moyen en volume de la phase aqueuse dispersée étant supérieur à 200 nm.

L'émulsion inverse conforme à l'invention permet d'obtenir rapidement un éclaircissement important des fibres kératiniques tout en limitant la dégradation des fibres kératiniques et l'irritation de la peau.

La présente invention a également pour objet un procédé de décoloration des fibres kératiniques mettant en oeuvre l'émulsion inverse ou la composition de décoloration prête à l'emploi conformes à l'invention, ainsi qu'un kit pour la mise en oeuvre de ce procédé.

Un autre objet de l'invention est l'utilisation de l'émulsion inverse ou de la composition de décoloration prête à l'emploi conformes à l'invention pour la décoloration des fibres kératiniques.

Dans le cadre de l'invention, on entend par émulsion inverse une émulsion à phase aqueuse dispersée.

Dans le cadre de la présente invention, on entend par phase inerte une phase qui est chimiquement inerte vis-à-vis du peroxyde d'hydrogène. Dans le cadre de l'invention, une phase est inerte si la dégradation du peroxyde d'hydrogène en présence de cette phase est inférieure à 25 % après 15 heures à 100 °C.

Dans le cadre de la présente invention, on entend par composé liquide non oxygéné et non perfluoré tout dérivé organique ou minéral non oxygéné et non perfluoré pouvant s'écouler et possédant une viscosité à 25 °C inférieure à 5 000 Poises à un taux de cisaillement de 1 S⁻¹. Cette viscosité peut être déterminée à l'aide d'un viscosimètre de type RHEOMAT 180. Le ou les composés liquides non oxygénés et non perfluorés sont choisis parmi les polyalphaoléfines, les polybutènes et les polyisobutènes, les huiles minérales, les huiles de paraffines, et leurs mélanges.

Selon un mode de réalisation particulier, la phase inerte représente entre 20 et 95 % en poids, et de préférence entre 30 et 90 % en poids du poids total de l'émulsion inverse.

Le diamètre moyen en volume de la phase aqueuse dispersée est de préférence compris entre 200 nm et 100 µm. Il peut être déterminé à l'aide d'un granulomètre laser tel que le granulomètre Master Sizer 2000 de la société MALVERN.

L'émulsion inverse conforme à l'invention présente une concentration en peroxyde d'hydrogène comprise entre 1 et 20 % en poids, de préférence entre 2 et 12 % en poids du poids total de l'émulsion inverse.

Selon un mode de réalisation particulier, l'émulsion inverse de l'invention comprend au moins un composé choisi parmi les agents tensioactifs anioniques, non ioniques, cationiques et amphotères, par exemple ceux possédant une ou plusieurs chaînes alkylées en C₆-C₂₂ linéaires ou ramifiées et / ou une ou plusieurs chaînes perfluorées, ainsi que les dérivés anioniques, non ioniques, amphotères et cationiques des anhydrides succiniques de polyisobutylène tels que décrits dans l'ouvrage J.M. Carey et al, Improved High Internal Phase Emulsions using Alkenyl Succinic Anhydride Based Emulsifiers, World Surfactants Congress, 5th, Firenze, Italy, May-June, 2000, 905-910, les polymères amphiphiles tels que les copolymères à blocs amphiphiles hydrosolubles ou hydrodispersibles, les polymères associatifs porteurs sur leur chaîne principale de groupements alkyle et / ou aryle en C₆-C₂₂, oxyéthylénés ou non, téléchéliques ou greffés, ces polymères pouvant être de charge cationique, anionique, amphotère ou non ionique, et les polymères épaississants tels que les polymères acryliques réticulés, les polysaccharides naturels ou modifiés.

Le ou les composés choisis parmi les dérivés des anhydrides succiniques de polyisobutylène, les polymères amphiphiles et les polymères épaississants sont chacun présents en une quantité comprise entre 0,1 et 30 % en poids, et de préférence entre 0,2 et 15 % en poids du poids total de l'émulsion inverse.

A titre de tensio-actifs utiles dans l'invention, on peut citer les tensioactifs anioniques non siliconés notamment les sels des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, a-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 12 à 20 atomes de carbone, et le radical aryle désignant de préférence un groupement phényle ou benzyle. Les sels de ces composés sont par exemple des sels alcalins, notamment de sodium, sels d'ammonium, sels d'amines, sels d'aminoalcools ou sels de magnésium. Parmi les tensioactifs anioniques on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides éthers carboxyliques polyoxyalkylénés et leurs sels, en particulier ceux comportant de 2 à 50 groupements oxyde d'éthylène, et leurs mélanges. Les tensioactifs anioniques du type acides ou sels d'éthers carboxyliques polyoxyalkylénés sont en particulier ceux qui répondent à la formule (1) suivante : 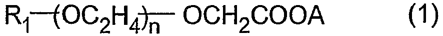 dans laquelle :
R₁ représente un groupement alkyle ou alkylaryle, et n est un nombre entier ou décimal (valeur moyenne) pouvant varier de 2 à 24 et de préférence de 3 à 10, le radical alkyle ayant entre 6 et 20 atomes de carbone environ, et aryle désignant de préférence phényle,
A représente un atome d'hydrogène, un ammonium, ion sodium, potassium, lithium ou magnésium ou un reste monoéthanolamine ou triéthanolamine.

On peut également utiliser des mélanges de composés de formule (1) en particulier des mélanges dans lesquels les groupements R₁ sont différents.

Parmi tous ces tensioactifs anioniques, on préfère utiliser les sels d'alkylsulfates et d'alkyléthersulfates, ainsi que leurs mélanges.

A titre de tensio-actifs amphotères non siliconés, on peut citer des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant par exemple carboxylate, sulfonate, sulfate, phosphate ou phosphonate ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits commercialisés sous les dénomination MIRANOL, tels que décrits dans les brevets US-2 528 378 et US-2 781 354 et de structures :

R₂ -CONHCH₂CH₂ -N(R₃)(R₄)(CH₂COO⁻) (2)

dans laquelle :
R₂ désigne un radical alkyle dérivé d'un acide R₂-COOH présent dans l'huile de coprah hydrolysée, un radical heptyle, nonyle ou undécyle,
R₃ désigne un groupement bêta-hydroxyéthyle ; et
R₄ un groupement carboxyméthyle ; et

R₅-CONHCH₂CH₂-N(B)(C) (3)

dans laquelle :
B représente -CH₂CH₂OX', C représente -(CH₂)_{z}-Y', avec z = 1 ou 2,
X' désigne le groupement -CH₂CH₂-COOH ou un atome d'hydrogène,
Y' désigne -COOH ou le radical -CH₂-CHOH-SO₃H,
R₅ désigne un radical alkyle d'un acide R₅-COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.

A titre d'exemple on peut citer le cocoamphodiacetate commercialisé sous la dénomination commerciale MIRANOL C2M concentré par la société RHONE POULENC.

A titre de tensioactif(s) non ionique(s) non siliconé(s), on peut citer les tensioactifs non-ioniques bien connus en soi (voir notamment à cet égard "Handbook of Surfactants" par M.R. PORTER, éditions Blackie & Son (Glasgow and London), 1991, pp 116-178). Ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀-C₁₄) amines ou les oxydes de N-acylaminopropylmorpholine. Selon un mode de réalisation de l'invention, les alkylpolyglycosides constituent des tensio-actifs non-ioniques utiles dans le cadre de la présente invention.

A titre de tensioactifs cationiques non siliconé, on peut utiliser les tensioactifs bien connus en soi, tels que les sels d'amines grasses primaires, secondaires ou tertiaires, éventuellement polyoxyalkylénées, les sels d'ammonium quaternaire, et leurs mélanges.

A titre de sels d'ammonium quaternaires, on peut notamment citer :
- ceux qui présentent la formule suivante : 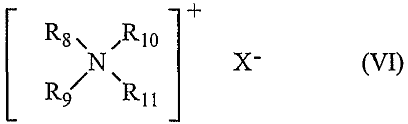 dans laquelle les radicaux R₈ à R₁₁, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre et les halogènes. Les radicaux aliphatiques sont par exemple choisis parmi les radicaux alkyle, alcoxy, polyoxyalkylène (C₂-C₆), alkylamide, alkyl(C₁₂-C₂₂)amidoalkyle(C₂-C₆), alkyl(C₁₂-C₂₂)acétate, hydroxyalkyle, comportant environ de 1 à 30 atomes de carbone ; X est un anion choisi dans le groupe des halogénures, phosphates, acétates, lactates, alkyl(C₂-C₆)sulfates, alkyl- ou alkylaryl-sulfonates ;
- les sels d'ammonium quaternaire de l'imidazoline, comme par exemple ceux de formule suivante : 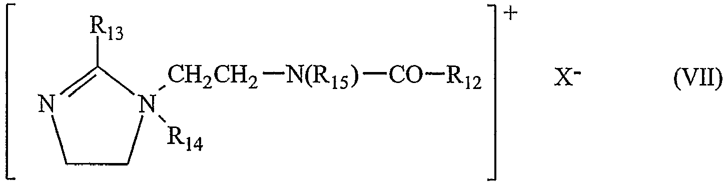 dans laquelle R₁₂ représente un radical alcényle ou alkyle comportant de 8 à 30 atomes de carbone, par exemple dérivés des acides gras du suif, R₁₃ représente un atome d'hydrogène, un radical alkyle en C₁-C₄ ou un radical alcényle ou alkyle comportant de 8 à 30 atomes de carbone, R₁₄ représente un radical alkyle en C₁-C₄, R₁₅ représente un atome d'hydrogène, un radical alkyle en C₁-C₄, X- est un anion choisi dans le groupe des halogénures, phosphates, acétates, lactates, alkylsulfates, alkyl- ou alkylaryl-sulfonates. De préférence, R₁₂ et R₁₃ désignent un mélange de radicaux alcényle ou alkyle comportant de 12 à 21 atomes de carbone, par exemple dérivés des acides gras du suif, R₁₄ désigne un radical méthyle, R₁₅ désigne un atome d'hydrogène. Un tel produit est par exemple commercialisé sous la dénomination REWOQUAT® W 75 par la société REWO ;
- les sels de diammonium quaternaire de formule (VIII) : 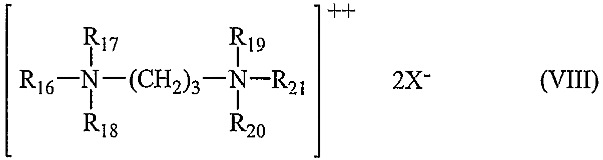 dans laquelle R₁₆ désigne un radical aliphatique comportant environ de 16 à 30 atomes de carbone, R₁₇, R₁₈, R₁₉, R₂₀ et R₂₁, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium ;
- les sels d'ammonium quaternaire contenant au moins une fonction ester, tels que ceux de formule (IX) suivante : 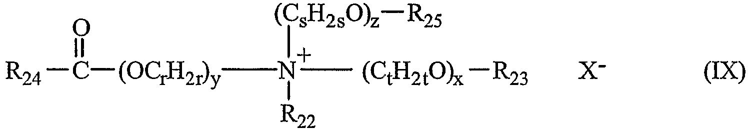 dans laquelle :
   R₂₂ est choisi parmi les radicaux alkyles en C₁-C₆ et les radicaux hydroxyalkyles ou dihydroxyalkyles en C₁-C₆ ;
   R₂₃ est choisi parmi :
      - le radical 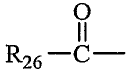
      - les radicaux R₂₇ hydrocarbonés en C₁-C₂₂, linéaires ou ramifiés, saturés ou insaturés,
      - l'atome d'hydrogène,
   R₂₅ est choisi parmi :
      - le radical 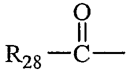
      - les radicaux R₂₉ hydrocarbonés en C₁-C₆, linéaires ou ramifiés, saturés ou insaturés,
      - l'atome d'hydrogène,
   R₂₄, R₂₆ et R₂₈, identiques ou différents, sont choisis parmi les radicaux hydrocarbonés en C₇-C₂₁, linéaires ou ramifiés, saturés ou insaturés ;
   r, s et t, identiques ou différents, sont des entiers valant de 2 à 6 ;
   y est un entier valant de 1 à 10 ;
   x et z, identiques ou différents, sont des entiers valant de 0 à 10 ;
   X⁻ est un anion simple ou complexe, organique ou inorganique ;
   sous réserve que la somme x + y + z vaut de 1 à 15, que lorsque x vaut 0 alors R₂₃ désigne R₂₇ et que lorsque z vaut 0 alors R₂₅ désigne R₂₉.

Les radicaux alkyles R₂₂ peuvent être linéaires ou ramifiés et plus particulièrement linéaires.

De préférence, R₂₂ désigne un radical méthyle, éthyle, hydroxyéthyle ou dihydroxypropyle, et plus particulièrement un radical méthyle ou éthyle.

Avantageusement, la somme x + y + z vaut de 1 à 10.

Lorsque R₂₃ est un radical R₂₇ hydrocarboné, il peut être long et avoir de 12 à 22 atomes de carbone, ou court et avoir de 1 à 3 atomes de carbone.

Lorsque R₂₅ est un radical R₂₉ hydrocarboné, il a de préférence 1 à 3 atomes de carbone.

Avantageusement, R₂₄, R₂₆ et R₂₈, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1.

Avantageusement, y est égal à 1.

De préférence, r, s et t, identiques ou différents, valent 2 ou 3, et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X- est encore plus particulièrement le chlorure ou le méthylsulfate.

On utilise plus particulièrement dans la composition selon l'invention, les sels d'ammonium de formule (IX) dans laquelle :
R₂₂ désigne un radical méthyle ou éthyle,
x et y sont égaux à 1 ;
z est égal à 0 ou 1 ;
r, s et t sont égaux à 2 ;
R₂₃ est choisi parmi :
   - le radical 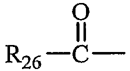
   - les radicaux méthyle, éthyle ou hydrocarbonés en C₁₄-C₂₂,
   - l'atome d'hydrogène ;
R₂₅ est choisi parmi :
   - le radical 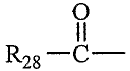
   - l'atome d'hydrogène ;
R₂₄, R₂₆ et R₂₈, identiques ou différents, sont choisis parmi les radicaux hydrocarbonés en C₁₃-C₁₇, linéaires ou ramifiés, saturés ou insaturés, et de préférence parmi les radicaux alkyles et alcényles en C₁₃-C₁₇, linéaires ou ramifiés, saturés ou insaturés.

Avantageusement, les radicaux hydrocarbonés sont linéaires.

On peut citer par exemple les composés de formule (IX) tels que les sels (chlorure ou méthylsulfate notamment) de diacyloxyéthyl-diméthylammonium, de diacyloxyéthylhydroxyéthylméthylammonium, de monoacyloxyéthyl-dihydroxyéthylméthylammonium, de triacyloxyéthylméthylammonium, de monoacyloxyéthylhydroxyéthyldiméthylammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthanesulfonate de méthyle, le para-toluènesulfonate de méthyle, la chlorhydrine du glycol ou du glycérol.

De tels composés sont par exemple commercialisés sous les dénominations DEHYQUART® par la société HENKEL, STEPANQUAT® par la société STEPAN, NOXAMIUM® par la société CECA, REWOQUAT® WE 18 par la société REWO-WITCO.

A titre de tensioactifs cationique, on préfère le mélange de sels de mono-, di- et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl-dihydroxyéthyl-méthylammonium, 45 à 60 % de méthylsulfate de diacyloxyéthyl-hydroxyéthyl-méthylammonium et 15 à 30 % de méthylsulfate de triacyloxyéthyl-méthylammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Parmi les sels d'ammonium quaternaire de formule (VI), on préfère d'une part, les chlorures de tétraalkylammonium comme, par exemple, les chlorures de dialkyldiméthylammonium ou d'alkyltriméthylammonium dans lesquels le radical alkyle comporte environ de 12 à 22 atomes de carbone, en particulier les chlorures de béhényltriméthylammonium, de distéaryldiméthylammonium, de cétyltriméthylammonium, de benzyldiméthylstéarylammonium ou encore, d'autre part, le chlorure de palmitylamidopropyltriméthylammonium ou le chlorure de stéaramidopropyldiméthyl-(myristyl acétate)-ammonium commercialisé sous la dénomination CERAPHYL® 70 par la société VAN DYK.

Les tensioactifs cationiques particulièrement préférés sont choisis parmi les sels d'ammonium quaternaire, et en particulier parmi le chlorure de béhényltriméthylammonium et le chlorure de palmitylamidopropyltriméthylammonium.

A titre de tensioactifs utilisables dans la présente invention on peut aussi particulièrement citer les tensioactifs siliconés décrits notamment dans le brevet FR 2 818 902. Les tensioactifs siliconés utilisables dans la présente invention sont ceux bien connus de l'homme du métier. Ils peuvent être non-ioniques, anioniques, cationiques ou amphotères. Ils peuvent être hydrosolubles, spontanément hydrodispersibles ou non hydrosolubles. De préférence, ils sont hydrosolubles ou spontanément hydrodispersibles.

Les tensioactifs siliconés sont, par exemple, choisis parmi les composés de formules générales suivantes : 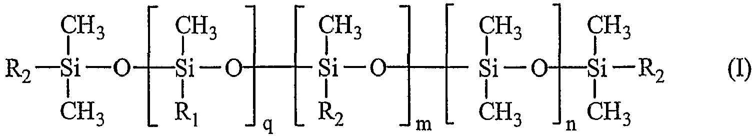 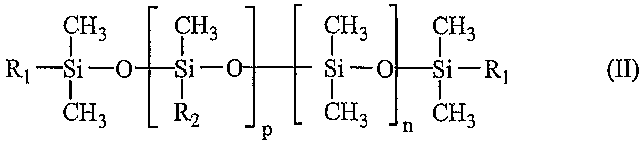 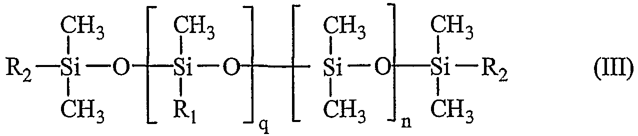 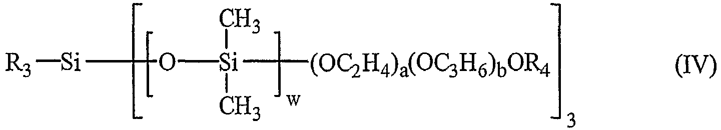 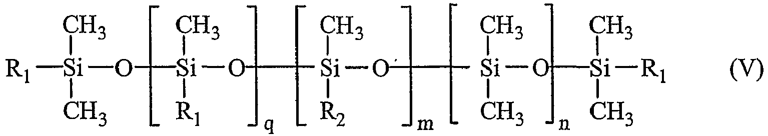 formules dans lesquelles :
R₁, identique ou différent, représente un groupe alkyle, linéaire ou ramifié, en C₁-C₃₀, ou phényle ;
R₂, identique ou différent, représente -C_{c}H_{2c}-O-(C₂H₄O)ₐ-(C₃H₆O)_{b}-R₅ ou -C_{c}H_{2c}-O-(C₄H₈O)ₐ-R₅ ;
R₃ et R₄, identiques ou différents, désignent chacun un groupe alkyle, linéaire ou ramifié, en C₁-C₁₂, et de préférence un groupe méthyle ;
R₅, identique ou différent, est choisi parmi un atome d'hydrogène, un groupe alkyle, linéaire ou ramifié, comportant de 1 à 12 atomes de carbone, un groupe alcoxy, linéaire ou ramifié, comportant de 1 à 6 atomes de carbone, un groupe acyle, linéaire ou ramifié, comportant de 2 à 12 atomes de carbone, un groupe hydroxyle, -SO₃M, -OCOR₆, aminoalcoxy en C₁-C₆ éventuellement substitué sur l'amine, aminoacyle en C₂-C₆ éventuellement substitué sur l'amine, -NHCH₂CH₂COOM, -N(CH₂CH₂COOM)₂, aminoalkyle en C₁-C₁₂ éventuellement substitué sur l'amine et sur la chaîne alkyle, carboxyacyle en C₁-C₃₀, un groupement phosphono éventuellement substitué par un ou deux groupes aminoalkyle en C₁-C₁₂ substitués, -CO(CH₂)_{d}COOM, -OCOCHR₇(CH₂)_{d}COOM, -NHCO(CH₂)_{d}OH, -NH₃Y ;
M, identique ou différent, désigne un atome d'hydrogène, Na, K, Li, NH₄ ou une amine organique ;
R₆ désigne un groupe alkyle, linéaire ou ramifié, en C₁-C₃₀,
R₇ désigne un atome d'hydrogène ou un groupe SO₃M ;
d varie de 1 à 10 ;
m varie de 0 à 20 ;
n varie de 0 à 500 ;
p varie de 1 à 50 ;
q varie de 0 à 20 ;
a varie de 0 à 50 ;
b varie de 0 à 50 ;
a + b est supérieur ou égal à 1 ;
c varie de 0 à 4 ;
w varie de 1 à 100 ;
Y représente un anion minéral ou organique monovalent tel qu'un halogénure (chlorure, bromure), un sulfate, ou un carboxylate (acétate, lactate, citrate).

De préférence, on utilise des tensioactifs siliconés répondant aux formules générales (IV) ou (VII) telles que définies ci-dessus, et plus particulièrement, ceux répondant aux formules (IV) ou (VII) dans lesquelles au moins l'une des, et de préférence toutes les conditions suivantes sont satisfaites :
c est égal à 2 ou 3 ;
R₁ désigne le groupe méthyle ;
R₅ représente un atome d'hydrogène, un groupe méthyle ou un groupe acétyle et de préférence un atome d'hydrogène ;
a varie de 1 à 25 et plus particulièrement de 2 à 25 ;
b varie de 0 à 25, de préférence de 10 à 20 ;
n varie de 0 à 100 ;
p varie de 1 à 20.

Les tensioactifs siliconés les plus particulièrement préférés sont, par exemple, ceux vendus sous les dénominations commerciales FLUID DC 193 et DC 5225C par la société DOW CORNING, SILWET® L 77 par la société OSI et MAZIL® 756 par la société MAZER PPG, le mélange LAURYL PEG / PPG-18 / 18 METHICONE (and) POLOXAMER 407 (and) DODECENE commercialisé par Dow Corning sous le nom de DC 5200.

Les tensioactifs siliconés les plus particulièrement préférés sont, par exemple, ceux vendus sous les dénominations commerciales FLUID DC 193 et DC 5225C par la société DOW CORNING, SILWET® L 77 par la société OSI et MAZIL® 756 par la société MAZER PPG, le mélange LAURYL PEG / PPG-18 / 18 METHICONE (and) POLOXAMER 407 (and) DODECENE commercialisé par Dow Corning sous le nom de DC 5200.

Les tensio-actifs présentant une HLB (hydrophilic Lipophilic Balance) inférieure à 10 sont préférés. En particulier, les tensioactifs non ioniques utilisés dans les compositions de l'invention présentent de préférence une HLB allant de 1,5 à 10, et mieux encore de 1,5 à 7. La HLB ou balance hydrophile-lipophile du ou des tensioactifs non ioniques utilisés selon l'invention est la HLB selon GRIFFIN définie dans la publication J. Soc. Cosm. Chem. 1954 (Volume 5), pages 249-256.

Selon un mode de réalisation préféré, le ou les tensio-actifs présents dans l'émulsion sont choisis parmi les tensio-actifs siliconés.

Les agents tensioactifs peuvent être chacun présents dans l'émulsion inverse conforme à l'invention en une quantité comprise entre 0,1 et 30 % en poids, et de préférence entre 0,2 et 15 % en poids du poids total de l'émulsion inverse.

La phase inerte de l'émulsion peut contenir un gélifiant / structurant spécifique comme les polyamides siliconés PSPA (DP100 et DP15) commercialisés par Dow Chemical, les KSG organiques polylauryldiméthylsiloxane et les KSG siliconés commercialisés par Shinetsu, le palmitate de dextrine et le stéarate d'inuline connu sous le nom de la gamme Rheopearl de Chiba Flour Milling) ; les acrylate à chaîne alkyle commercialisé par Landec, les copolymères éthylène octène commercialisé par Dupont de Nemours, le dibutyl lauryl glutamide commercialisé par Ajinomoto, le disorbène commercialisé par Roquette, les copolymères styrène acrylate Versagel M5960 et 5670 commercialisés par Penreco, les kratons dibloc et tribloc commercialisés par Kraton Polymers, l'acide hydroxystéariques, les cires de jojoba, les silicices pyrogénées commercialisées par Degussa, les cires de silicones commercialisés par Waker, le polyamide Uniclear commercialisé par Arizona Chemical, la bentone.

La phase inerte peut comprendre d'autres huiles que les composés liquides non oxygénés et non perfluorés.

La phase aqueuse de l'émulsion inverse conforme à l'invention présente un pH inférieur à 5. Le pH peut être ajusté à la valeur désirée au moyen d'agents acides ou alcalins habituellement utilisés en cosmétique ou bien encore à l'aide de systèmes tampons classiques.

Parmi les agents acides, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalins on peut citer, à titre d'exemple, l'ammoniaque, les carbonates alcalins, les alcanolamines telles que les di-et triéthanolamines ainsi que leurs dérivés, les hydroxydes de sodium ou de potassium et les composés de formule (X) suivante : 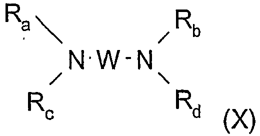 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

Afin de décolorer les fibres kératiniques, l'émulsion inverse conforme à l'invention peut être appliquée telle quelle sur les fibres kératiniques. Elle peut aussi être mélangée au moment de l'emploi avec une ou plusieurs compositions dans lesquelles sont répartis au moins un agent alcalin et / ou au moins un persel et / ou au moins un colorant direct et / ou au moins un précurseur de colorant d'oxydation, pour conduire à une composition de décoloration prête à l'emploi.

Dans le cas où la composition de décoloration prête à l'emploi comprend au moins un colorant direct et / ou au moins un précurseur de colorant d'oxydation, on réalise une coloration éclaircissante, c'est-à-dire que l'on obtient simultanément une décoloration et une coloration des fibres kératiniques.

Le ou les agents alcalins peuvent être choisis parmi les amines organiques, l'ammoniaque et les silicates.

Lorsque la composition de décoloration prête à l'emploi conforme à l'invention comprend un ou plusieurs agents alcalins, ils sont généralement présents en quantité comprise entre 0,01 et 40 % en poids, et de préférence entre 0,1 et 30 % en poids du poids total de la composition prête à l'emploi.

Le ou les persels peuvent être choisis parmi les perborates, les percarbonates et les persulfates, d'ammonium ou de métaux alcalins.

Lorsque la composition de décoloration prête à l'emploi conforme à l'invention comprend un ou plusieurs persels, ils sont généralement présents en quantité comprise entre 10 et 70 % en poids, et de préférence entre 20 et 60 % en poids du poids total de la composition prête à l'emploi.

Le ou les colorants directs peuvent être choisis parmi les colorants directs classiquement utilisés en coloration directe. A titre d'exemples, ces colorants directs sont choisis parmi les colorants nitrés de la série benzénique, les colorants directs azoïques, les colorants directs méthiniques, les colorants directs quinoniques, les colorants directs aziniques, les colorants directs triarylméthaniques, les colorants directs indoaminiques, les colorants directs naturels. Ces colorants directs peuvent être de nature non ionique, anionique ou cationique.

Parmi les colorants directs benzéniques, on peut citer le 1,4-diamino-2-nitrobenzène, le 1-amino-2-nitro-4-(β-hydroxyéthylamino)-benzène, le 1-amino-2-nitro-4-bis(β-hydroxyéthyl)-aminobenzène, le 1,4-bis(β-hydroxyéthylamino)-2-nitro-benzène, le 1-p-hydroxyéthylamino-2-nitro-4-bis-(β-hydroxyéthylamino)-benzène, le 1-β-hydroxyéthylamino-2-nitro-4-amino-benzène, le 1-β-hydroxyéthylamino-2-nitro-4-(éthyl)(β-hydroxyéthyl)-aminobenzène, le 1-amino-3-méthyl-4-p-hydroxyéthylamino-6-nitro-benzène, le 1-amino-2-nitro-4-β-hydroxyéthylamino-5-chloro-benzène, le 1,2-diamino-4-nitro-benzène, le 1-amino-2-β-hydroxyéthylamino-5-nitro-benzène, le 1,2-bis-(β-hydroxyéthylamino)-4-nitro-benzène, le 1-amino-2-[tris-(hydroxyméthyl)-méthylamino]-5-nitrobenzène, le 1-hydroxy-2-amino-5-nitro-benzène, le 1-hydroxy-2-amino-4-nitro-benzène, le 1-hydroxy-3-nitro-4-amino-benzène, le 1-hydroxy-2-amino-4,6-dinitro-benzène, le 1-β-hydroxyéthyloxy-2-β-hydroxyéthylamino-5-nitro-benzène, le 1-méthoxy-2-β-hydroxyéthylamino-5-nitro-benzène, le 1-β-hydroxyéthyloxy-3-méthylamino-4-nitrobenzène, le 1-β,γ-dihydroxypropyloxy-3-méthylamino-4-nitro-benzène, le 1-β-hydroxyéthylamino-4-β,γ-dihydroxypropyloxy-2-nitro-benzène, le 1-β,γ-dihydroxypropylamino-4-trifluorométhyl-2-nitro-benzène, le 1-β-hydroxyéthylamino-4-trifluorométhyl-2-nitro-benzène, le 1-β-hydroxyéthylamino-3-méthyl-2-nitro-benzène, le 1-β-aminoéthylamino-5-méthoxy-2-nitro-benzène, le 1-hydroxy-2-chloro-6-éthylamino-4-nitrobenzène, le 1-hydroxy-2-chloro-6-amino-4-nitro-benzène, le 1-hydroxy-6-[bis-(β-hydroxyéthyl)-amino]-3-nitro-benzène, le 1-p-hydroxyéthylamino-2-nitro-benzène, le 1-hydroxy-4-β-hydroxyéthylamino-3-nitro-benzène.

Parmi les colorants directs azoïques, on peut citer les colorants azoïques cationiques décrits dans les demandes de brevets WO 95/15144, WO-95/01772, EP 0 714 954, FR 2 822 696, FR 2 825 702, FR 2 825 625, FR 2 822 698, FR 2 822 693, FR 2 822 694, FR 2 829 926, FR 2 807 650, WO 02/078660, WO 02/100834, WO 02/100369 et FR 2 844 269 dont le contenu fait partie intégrante de l'invention.

Parmi ces composés, on peut tout particulièrement citer le chlorure de 1,3-diméthyl-2-[[4-(diméthylamino)phényl]azo]-1H-Imidazolium, le chlorure de 1,3-diméthyl-2-[(4-aminophényl)azo]-1H-Imidazolium, le méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3^{e} édition : Disperse Red 17, Acid Yellow 9, Acid Black 1, Basic Red 22, Basic Red 76, Basic Yellow 57, Basic Brown 16, Acid Yellow 36, Acid Orange 7, Acid Red 33, Acid Red 35, Basic Brown 17, Acid Yellow 23, Acid Orange 24, Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques, on peut citer les colorants suivants: Disperse Red 15, Solvent Violet 13, Acid Violet 43, Disperse Violet 1, Disperse Violet 4, Disperse Blue 1, Disperse Violet 8, Disperse Blue 3, Disperse Red 11, Acid Blue 62, Disperse Blue 7, Basic Blue 22, Disperse Violet 15, Basic Blue 99, ainsi que les composés suivants : la 1-N-méthylmorpholiniumpropylamino-4-hydroxyanthraquinone, la 1-aminopropylamino-4-méthylaminoanthraquinone, la 1-aminopropylaminoanthraquinone, la 5-β-hydroxyéthyl-1,4-diaminoanthraquinone, la 2-aminoéthylaminoanthraquinone, la 1,4-bis-(β,?-dihydroxypropylamino)-anthraquinone.

Parmi les colorants aziniques, on peut citer les composés suivants : Basic Blue 17, Basic Red 2.

Parmi les colorants triarylméthaniques, on peut citer les composés suivants : Basic Green 1, Acid blue 9, Basic Violet 3, Basic Violet 14, Basic Blue 7, Acid Violet 49, Basic Blue 26, Acid Blue 7.

Parmi les colorants indoaminiques, on peut citer les composés suivants : la 2-β-hydroxyéthlyamino-5-[bis-(β-4'-hydroxyéthyl)amino]anilino-1,4-benzoquinone, la 2-β-hydroxyéthylamino-5-(2'-méthoxy-4'-amino)anilino-1,4-benzoquinone, la 3-N(2'-Chloro-4'-hydroxy)phényl-acétylamino-6-méthoxy-1,4-benzoquinone imine, la 3-N(3'-Chloro-4'-méthylamino)phényl-uréido-6-méthyl-1,4-benzoquinone imine, la 3-[4'-N-(Ethyl,carbamylméthyl)-amino]-phényl-uréido-6-méthyl-1,4-benzoquinone imine.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Lorsque la composition de décoloration prête à l'emploi conforme à l'invention comprend un ou plusieurs colorants directs, ils sont généralement présents en quantité comprise entre 0,001 et 20 % en poids environ du poids total de la composition prête à l'emploi, et encore plus préférentiellement entre 0,005 et 10 % en poids environ.

Les précurseurs de colorants d'oxydation peuvent être choisis parmi les bases d'oxydation et les coupleurs conventionnellement utilisés dans le domaine de la coloration.

A titre d'exemples de bases d'oxydation, on peut citer les para-phénylènediamines, les bases doubles, les para-aminophénols, les ortho-aminophénols, les bases hétérocycliques et leurs sels d'addition.

Parmi les para-phénylènediamines, on peut citer à titre d'exemple, la para-phénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylène-diamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine et leurs sels d'addition avec un acide.

Parmi les para-phénylènediamines citées ci-dessus, la para-phénylènediamine, la para-toluènediamine, la 2-isopropyl para-phénylènediamine, la 2-β-hydroxyéthyl para-phénylènediamine, la 2-β-hydroxyéthyloxy para-phénylène-diamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Parmi les bases doubles, on peut citer, à titre d'exemples, les bis-phénylalkylènediamines et les bis-para-aminophénols.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition avec un acide ou avec une base.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2750048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Lorsque la composition de décoloration prête à l'emploi conforme à l'invention comprend une ou plusieurs bases d'oxydation, elles sont généralement présentes en quantité comprise entre 0,001 et 10 % en poids environ du poids total de la composition prête à l'emploi, de préférence entre 0,005 et 6 % en poids environ.

A titre d'exemples de coupleurs, on peut citer les méta-phénylènediamines, les méta-aminophénols, les méta-diphénols, les coupleurs naphtaléniques, les coupleurs hétérocycliques et leurs sels d'addition.

A titre d'exemples, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Lorsque la composition de décoloration prête à l'emploi conforme à l'invention comprend un ou plusieurs coupleurs, ils sont généralement présents en quantité comprise entre 0,001 et 10 % en poids environ du poids total de la composition prête à l'emploi, de préférence entre 0,005 et 6 % en poids environ.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telle que la soude, la potasse, l'ammoniaque, les amines ou les alcanolamines.

Selon un mode de réalisation particulier, la composition de décoloration prête à l'emploi conforme à l'invention comprend au moins un agent alcalin tel que défini précédemment. Elle peut alors résulter du mélange de l'émulsion inverse conforme à l'invention avec une composition aqueuse comprenant, dans un milieu cosmétique approprié, le ou les agents alcalins.

Selon un autre mode de réalisation particulier, la composition de décoloration prête à l'emploi conforme à l'invention comprend au moins un agent alcalin et au moins un persel tels que définis précédemment. Elle peut alors résulter du mélange de l'émulsion inverse conforme à l'invention avec une composition anhydre comprenant, dans un milieu cosmétique approprié, le ou les agents alcalins et le ou les persels. Elle peut aussi résulter du mélange de l'émulsion inverse conforme à l'invention avec une composition aqueuse comprenant, dans un milieu cosmétique approprié, le ou les agents alcalins et une composition anhydre comprenant, dans un milieu cosmétique approprié, le ou les persels.

Selon un autre mode de réalisation particulier, la composition de décoloration prête à l'emploi conforme à l'invention comprend au moins un colorant direct tel que défini précédemment. Elle peut alors résulter du mélange de l'émulsion inverse conforme à l'invention avec une composition aqueuse comprenant, dans un milieu cosmétique approprié, le ou les colorants directs.

Selon un autre mode de réalisation particulier, la composition de décoloration prête à l'emploi conforme à l'invention comprend au moins un agent alcalin et au moins un colorant direct et / ou au moins un précurseur de colorant d'oxydation tels que définis précédemment. Elle peut alors résulter du mélange de l'émulsion inverse conforme à l'invention avec une composition aqueuse comprenant, dans un milieu cosmétique approprié, le ou les agents alcalins et le ou les colorants directs et / ou le ou les précurseurs de colorant d'oxydation. Elle peut également résulter du mélange de l'émulsion inverse conforme à l'invention avec une composition aqueuse comprenant, dans un milieu cosmétique approprié, le ou les agents alcalins et une composition aqueuse comprenant, dans un milieu cosmétique approprié, le ou les colorants directs et / ou le ou les précurseurs de colorant d'oxydation.

Selon un autre mode de réalisation particulier, la composition de décoloration prête à l'emploi conforme à l'invention comprend au moins un agent alcalin, au moins un persel et au moins un colorant direct tels que définis précédemment. Elle peut alors résulter du mélange de l'émulsion inverse conforme à l'invention avec une composition anhydre comprenant, dans un milieu cosmétique approprié, le ou les agents alcalins et le ou les persels et une composition aqueuse comprenant, dans un milieu cosmétique approprié, le ou les colorants directs. Elle peut aussi résulter du mélange de l'émulsion inverse conforme à l'invention avec une composition aqueuse comprenant, dans un milieu cosmétique approprié, le ou les agents alcalins, une composition anhydre comprenant, dans un milieu cosmétique approprié, le ou les persels et une composition aqueuse comprenant, dans un milieu cosmétique approprié, le ou les colorants directs.

Les compositions comprenant le ou les persels sont anhydres. Elles peuvent également comprendre des additifs usuels dans le domaine, en particulier des polymères épaississants hydrosolubles, des charges telles que des argiles ou de la silice amorphe, des liants tels que la vinylpyrrolidone, des lubrifiants comme les stéarates de polyol ou les stéarates de métaux alcalins ou alcalino-terreux, ainsi que des agents de contrôle du dégagement d'oxygène tels que le carbonate ou l'oxyde de magnésium, des agents colorants ou des agents matifiants comme les oxydes de titane ou encore des agents tensioactifs anioniques, non ioniques, cationiques ou amphotères, des vitamines.

A titre illustratif, la teneur en additif(s) représentent 0,01 à 40 % en poids, de préférence de 0,1 à 30 % en poids par rapport au poids total des compositions.

Les compositions anhydres peuvent se présenter sous forme de poudre ou de pâte. Dans le cas où elles se présentent sous forme de pâte, elles comprennent de plus un liquide inerte organique choisi parmi les polydécènes de formule C₁₀ₙH_{[(20n)+2]} dans laquelle n varie de 3 à 9 et de préférence de 3 à 7, les esters d'alcools gras ou d'acides gras, les esters ou di-esters de sucres d'acides gras en C₁₂-C₂₄, les éthers cycliques ou les esters cycliques, les huiles de silicone, les huiles minérales ou les huiles végétales.

Les autres compositions sont de préférence aqueuses. Le milieu cosmétique approprié de ces compositions est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids par rapport au poids total de la composition, et plus préférentiellement encore entre 5 et 30 % en poids environ.

Ces compositions peuvent également renfermer divers adjuvants utilisés classiquement dans les compositions de décoloration des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci-dessus peuvent être présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à ces compositions ne soient pas, ou substantiellement pas, altérées par la ou les adjonctions envisagées.

Ces compositions peuvent se présenter sous des formes diverses, telles que sous forme de liquides, de crèmes, de gels, ou sous toute autre forme cosmétique appropriée.

Le pH de la composition de décoloration prête à l'emploi conforme à l'invention est compris entre 4 et 11. Il est préférentiellement alcalin, et encore plus préférentiellement compris entre 7 et 11.

Le procédé de décoloration conforme à la présente invention consiste à appliquer sur les fibres kératiniques une émulsion inverse. La présente invention a également pour objet un kit pour la décoloration des fibres kératiniques contenant une émulsion inverse conforme à l'invention. La présente invention a aussi pour objet l'utilisation pour la décoloration des fibres kératiniques d'une émulsion inverse ou d'une composition de décoloration prête à l'emploi conformes à l'invention telles que définies précédemment.

Les exemples qui suivent servent à illustrer l'invention sans toutefois présenter un caractère limitatif.

### EXEMPLE

On a préparé la composition oxydante 1 suivante :

| **COMPOSITION OXYDANTE 1** | |
|---|---|
| Huile de paraffine | 40 g |
| Arlacel P135 | 4 g |
| Arlamol ISML | 1 g |
| Ammonium acryloyldimethyltaurate / steareth-25 methacrylate crosspolymer | 2 g |
| Sulfate de magnésium | 0,3 g |
| Peroxyde d'hydrogène | 6 g |
| Stannate de sodium, 6 H₂O | 0,04 g |
| Acide diéthylène triamine pentacétique | 0,015 g |
| Pyrophosphate tétrasodique, 10 H₂O | 0,03 g |
| Acide phosphorique | qsp pH = 3 |
| Eau déminéralisée | qsp 100 g |

La composition oxydante 1 est mélangée au moment de l'emploi à une poudre décolorante contenant 50 % de persulfates, 24,1 % de silicates et 2,6 % de chlorure d'ammonium, avec un rapport poudre décolorante / composition oxydante égal à 1 / 1,5. On obtient le mélange A.

A titre de référence, une composition oxydante à 6 % de peroxyde d'hydrogène et de pH égal à 2 est mélangée au moment de l'emploi à la même poudre décolorante avec un rapport poudre décolorante / composition oxydante égal à 1 / 1,5. On obtient le mélange B.

Les mélanges obtenus sont appliqués sur des mèches de cheveux naturels châtains de 2,5 g, à raison de 10 g de mélange pour 1 g de cheveux. Après un temps de pose de 40 minutes, les mèches sont rincées, lavées avec un shampooing standard, rincées à nouveau puis séchées.

Les résultats sont décrits dans le tableau ci-dessous.

| **COMPARAISON DES NIVEAUX DE DECOLORATION** | | |
|---|---|---|
| | Mélange A (invention) | Mélange B (art antérieur) |
| Effet décolorant | HT = 9,25 | HT = 8,5 |
| Hauteur de ton des cheveux naturels châtains initiaux : HT = 4 | | |

On constate que la composition oxydante conforme à l'invention permet d'obtenir un niveau d'éclaircissement plus important que la composition oxydante connue de l'art antérieur.

## Revendications

1. Emulsion inverse pour la décoloration des fibres kératiniques comprenant une phase inerte comprenant au moins un composé liquide non oxygéné et non perfluoré présentant une solubilité dans l'eau à 25 °G inférieure à 1 %, choisi parmi les polyalphaoléfines, les polybutènes et les polyisobutènes, les huiles minérales, les huiles de paraffines, et leurs mélanges, et une solution aqueuse de peroxyde d'hydrogène, la concentration en peroxyde d'hydrogène étant comprise entre 1 et 20 % en poids du poids total de l'émulsion inverse,
la phase inerte représentant au moins 20 % en poids du poids total de l'émulsion inverse et le diamètre moyen en volume de la phase aqueuse dispersée étant supérieur à 200 nm.

2. Emulsion inverse selon la revendication 1, dans laquelle la phase inerte représente entre 20 et 95 % en poids du poids total de l'émulsion inverse.

3. Emulsion inverse selon la revendication 2, dans laquelle la phase inerte représente entre 30 et 90 % en poids du poids total de l'émulsion inverse.

4. Emulsion inverse selon l'une quelconque des revendications précédentes, dans laquelle le diamètre moyen en volume de la phase aqueuse dispersée est compris entre 200 nm et 100 µm.

5. Emulsion inverse selon l'une quelconque des revendications précédentes, dans laquelle la concentration en peroxyde d'hydrogène est comprise entre 2 et 12 % en poids du poids total de l'émulsion inverse.

6. Emulsion inverse selon l'une quelconque des revendications précédentes, comprenant au moins un composé choisi parmi les agents tensioactifs, les dérivés des anhydrides succiniques de polyisobutylène, les polymères amphiphiles et les polymères épaississants.

7. Emulsion inverse selon la revendication 6, dans laquelle le ou les agents tensioactifs sont choisis parmi les agents tensioactifs anioniques, non ioniques, cationiques et amphotères, possédant une ou plusieurs chaînes alkylées en C₆-C₂₂ linéaires ou ramifiées et / ou une ou plusieurs chaînes perfluorées.

8. Emulsion inverse selon la revendication 6 ou 7, dans laquelle le ou les agents tensioactifs sont choisis parmi les agents tensioactifs siliconés.

9. Emulsion inverse selon l'une quelconque des revendications 6 à 8, dans laquelle le ou les agents tensioactifs sont chacun présents en une quantité comprise entre 0,1 % et 30 % en poids du poids total de l'émulsion inverse.

10. Emulsion inverse selon l'une quelconque des revendications 6 à 9, dans laquelle le ou les dérivés des anhydrides succiniques de polyisobutylène sont anioniques, non ioniques, amphotères ou cationiques.

11. Emulsion inverse selon l'une quelconque des revendications 6 à 10, dans laquelle le ou les polymères amphiphiles sont choisis parmi les copolymères à blocs amphiphiles hydrosolubles ou hydrodispersibles, les polymères associatifs porteurs sur leur chaîne principale de groupements alkyle et / ou aryle en C₆-C₂₂, oxyéthylénés ou non, téléchéliques ou greffés, ces polymères pouvant être de charge cationique, anionique, amphotère ou non ionique.

12. Emulsion inverse selon l'une quelconque des revendications 6 à 11, dans laquelle le ou les polymères épaississants sont choisis parmi les polymères acryliques réticulés, les polysaccharides naturels ou modifiés.

13. Emulsion inverse selon l'une quelconque des revendications 6 à 12, dans laquelle le ou les composés choisis parmi les dérivés des anhydrides succiniques de polyisobutylène, les polymères amphiphiles et les polymères épaississants sont chacun présents en une quantité comprise entre 0,1 et 30 % en poids du poids total de l'émulsion inverse.

14. Emulsion inverse selon l'une quelconque des revendications précédentes, dont le pH de la phase aqueuse est inférieur à 5.

15. Procédé de décoloration des fibres kératiniques, **caractérisé en ce que** l'on applique sur les fibres kératiniques une émulsion inverse telle que définie à l'une quelconque des revendications 1 à 14.

16. Kit pour la décoloration des fibres kératiniques contenant une émulsion inverse telle que définie à l'une quelconque des revendication 1 à 14.

17. Utilisation pour la décoloration des fibres kératiniques d'une émulsion inverse telle que définie à l'une quelconque des revendications 1 à 14.

## Patentansprüche

1. Inverse Emulsion zum Bleichen von Keratinfasern, umfassend eine inerte Phase, die mindestens eine keinen Sauerstoff enthaltende und nicht perfluorierte flüssige Verbindung mit einer Wasserlöslichkeit bei 25 °C von weniger als 1 %, die aus Poly-alpha-olefinen, Polybutenen und Polyisobutenen, Mineralölen, Paraffinölen und Mischungen davon ausgewählt ist, und eine wässrige Lösung von Wasserstoffperoxid, wobei die Wasserstoffperoxid-Konzentration zwischen 1 und 20 Gew.-% des Gesamtgewichts der inversen Emulsion beträgt, wobei die inerte Phase mindestens 20 Gew.-% des Gesamtgewichts der inversen Emulsion ausmacht und der volumenmittleren Durchmesser der dispersen wässrigen Phase über 200 nm liegt.

2. Inverse Emulsion nach Anspruch 1, wobei die inerte Phase zwischen 20 und 95 Gew.-% des Gesamtgewichts der inversen Emulsion ausmacht.

3. Inverse Emulsion nach Anspruch 2, wobei die inerte Phase zwischen 30 und 90 Gew.-% des Gesamtgewichts der inversen Emulsion ausmacht.

4. Emulsion nach einem der vorhergehenden Ansprüche, wobei der volumenmittlere Durchmesser der dispersen wässrigen Phase zwischen 200 nm und 100 µm liegt.

5. Inverse Emulsion nach einem der vorhergehenden Ansprüche, wobei die Wasserstoffperoxid-Konzentration zwischen 2 und 12 Gew.-% des Gesamtgewichts der inversen Emulsion liegt.

6. Inverse Emulsion nach einem der vorhergehenden Ansprüche, umfassend mindestens eine Verbindung, die aus Tensiden, Derivaten von Polyisobutylenbernsteinsäureanhydriden, amphiphilen Polymeren und verdickenden Polymeren ausgewählt ist.

7. Inverse Emulsion nach Anspruch 6, wobei das Tensid bzw. die Tenside aus anionischen, nichtionischen, kationischen und amphoteren Tensiden mit einer oder mehreren linearen oder verzweigten C₆-C₂₂-Alkylketten und/oder einer oder mehreren perfluorierten Ketten ausgewählt ist bzw. sind.

8. Inverse Emulsion nach Anspruch 6 oder 7, wobei das Tensid bzw. die Tenside aus Silikon-Tensiden ausgewählt ist bzw. sind.

9. Inverse Emulsion nach einem der Ansprüche 6 bis 8, wobei das Tensid bzw. die Tenside jeweils in einer Menge zwischen 0,1 und 30 Gew.-% des Gesamtgewichts der inversen Emulsion vorliegt bzw. vorliegen.

10. Inverse Emulsion nach einem der Ansprüche 6 bis 9, wobei das Derivat bzw. die Derivate von Polyisobutylenbernsteinsäureanhydriden anionisch, nichtionisch, amphoter oder kationisch sind.

11. Inverse Emulsion nach einem der Ansprüche 6 bis 10, wobei das amphiphile Polymer bzw. die amphiphilen Polymere aus wasserlöslichen öder wasserdispergierbaren amphiphilen Blockcopolymeren und oxyethylenierten oder nicht oxyethylenierten, telechelen oder gepfropften assoziativen Polymeren, die an ihrer Hauptkette C₆-C₂₂-Alkyl- oder -Arylgruppen tragen, ausgewählt ist bzw. sind, wobei diese Polymere kationische, anionische, amphotere oder nichtionische Ladung aufweisen können.

12. Inverse Emulsion nach einem der Ansprüche 6 bis 11, wobei das verdickende Polymer bzw. die verdickenden Polymere aus vernetzten Acrylpolymeren und natürlichen oder modifizierten Polysacchariden ausgewählt ist bzw. sind.

13. Inverse Emulsion nach einem der Ansprüche 6 bis 12, wobei die Verbindung bzw. die Verbindungen, die aus Derivaten von Polyisobutylenbernsteinsäureanhydriden, amphiphilen Polymeren und verdickenden Polymeren ausgewählt ist bzw. sind, jeweils in einer Menge zwischen 0,1 und 30 Gew.-% des Gesamtgewichts der inversen Emulsion vorliegt bzw. vorliegen.

14. Inverse Emulsion nach einem der vorhergehenden Ansprüche, wobei der pH-Wert der wässrigen Phase unter 5 liegt.

15. Verfahren zum Bleichen von Keratinfasern, **dadurch gekennzeichnet, dass** man auf die Keratinfasern eine inverse Emulsion gemäß einem der Ansprüche 1 bis 14 aufbringt.

16. Kit zum Bleichen von Keratinfasern, das eine inverse Emulsion gemäß einem der Ansprüche 1 bis 14 enthält.

17. Verwendung einer inversen Emulsion gemäß einem der Ansprüche 1 bis 14 zum Bleichen von Keratinfasern.

## Claims

1. Invert emulsion for bleaching keratin fibres, comprising an inert phase comprising at least one nonoxygenated and nonperfluorinated liquid compound having a water-solubility at 25°C of less than 1%, chosen from polyalphaolefins, polybutenes and polyisobutenes, mineral oils, paraffin oils, and mixtures thereof, and an aqueous hydrogen peroxide solution, the hydrogen peroxide concentration being between 1 and 20% by weight of the total weight of the invert emulsion,
the inert phase representing at least 20% by weight of the total weight of the invert emulsion and the mean diameter by volume of the dispersed aqueous phase being greater than 200 nm.

2. Invert emulsion according to Claim 1, in which the inert phase represents between 20 and 95% by weight of the total weight of the invert emulsion.

3. Invert emulsion according to Claim 2, in which the inert phase represents between 30 and 90% by weight of the total weight of the invert emulsion.

4. Invert emulsion according to any one of the preceding claims, in which the mean diameter by volume of the dispersed aqueous phase is between 200 nm and 100 µm.

5. Invert emulsion according to any one of the preceding claims, in which the hydrogen peroxide concentration is between 2 and 12% by weight of the total weight of the invert emulsion.

6. Invert emulsion according to any one of the preceding claims, comprising at least one compound chosen from surfactants, derivatives of polyisobutylene succinic anhydrides, amphiphilic polymers and thickening polymers.

7. Invert emulsion according to Claim 6, in which the surfactant(s) are chosen from anionic, nonionic, cationic and amphoteric surfactants having one or more linear or branched C₆-C₂₂ alkylated chains and/or one or more perfluorinated chains.

8. Invert emulsion according to Claim 6 or 7, in which the surfactant(s) are chosen from silicone surfactants.

9. Invert emulsion according to any one of Claims 6 to 8, in which the surfactant(s) are each present in a quantity of between 0.1% and 30% by weight of the total weight of the invert emulsion.

10. Invert emulsion according to any one of Claims 6 to 9 in which the derivatives of polyisobutylene succinic anhydrides are anionic, nonionic, amphoteric or cationic.

11. Invert emulsion according to any one of Claims 6 to 10, in which the amphiphilic polymer(s) are chosen from water-soluble or water-dispersible amphiphilic block copolymers, oxyethylenated or nonoxyethylenated, telechelic or graft associative polymers bearing on their main chain C₆-C₂₂ alkyl and/or aryl groups, it being possible for these polymers to have a cationic, anionic, amphoteric or nonionic charge.

12. Invert emulsion according to any one of Claims 6 to 11, in which the thickening polymer(s) are chosen from crosslinked acrylic polymers, natural or modified polysaccharides.

13. Invert emulsion according to any one of Claims 6 to 12, in which the compound(s) chosen from derivatives of polyisobutylene succinic anhydrides, amphiphilic polymers and thickening polymers are each present in a quantity of between 0.1 and 30% by weight of the total weight of the invert emulsion.

14. Invert emulsion according to any one of the preceding claims, the pH of the aqueous phase of which is less than 5.

15. Method for bleaching keratin fibres, **characterized in that** an invert emulsion as defined in any one of Claims 1 to 14.

16. Kit for bleaching keratin fibres, containing an invert emulsion as defined in any one of Claims 1 to 14.

17. Use, for bleaching keratin fibres, of an invert emulsion as defined in any one of Claims 1 to 14.
